# EUROPEAN PATENT SPECIFICATION

(11) **EP 2 661 258 B1**
(45) Date of publication and mention of the grant of the patent: **27.04.2016**
(21) Application number: 12706950.8
(22) Date of filing: 06.01.2012
(51) Int. Cl.: A61K 31/675, A61K 9/16, A61K 9/20

(54) **IMPROVED RISEDRONATE FORMULATION**
VERBESSERTE RISEDRONAT-FORMULIERUNG
FORMULATION AMÉLIORÉE À BASE DE RISÉDRONATE

(30) Priority: 06.01.2011 TR 201100149
(43) Date of publication of application: 13.11.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2012/000011
(87) International publication number: WO 2012/093980

(56) References cited:
- WO-A1-2010/090614

## Description

### Technical Field

The present invention relates to highly bioavailable formulations comprising risedronate and/or a pharmaceutically suitable derivative thereof which protect epithelial and mucosal layer of the mouth, buccal cavity, pharynx, larynx and esophagus from erosion, ulcer and similar irritations in order to be used in prevention and treatment of bone loss and diseases associated with bone loss.

### The prior art

Bone loss or in other words "osteoporosis" is defined as a disease characterized by low bone mass and susceptibility to bone fragility as a result of the distortion in microstructure of bone tissue and increased risk in bone fracture. The disease does not result in death; however, it is a major health problem affecting quality of life in the world.

Risedronic acid *(Formula I)* is a bisphosphonate which has the chemical name of [1-hydroxy-2-(3-piridinyl) ethylidene] bisphosphonic acid. Risedronic acid was first disclosed in the patent numbered US 5583122 and the processes in order to prepare risedronic acid and use of risedronic acid in treatment of diseases associated with abnormal calcium and phosphate metabolism are included in this patent.

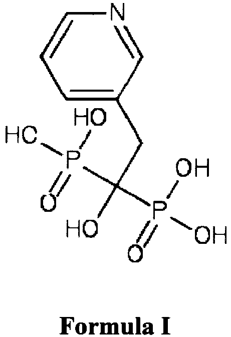

The active agents, particularly bisphosphonates, which are used in prevention and treatment of bone loss and diseases associated with bone loss, should be administered for a long period of time in terms of efficiency of the treatment. It has been seen that use of active agents which are used in treatment of such diseases by the oral route is more advantageous for patient compliance with the treatment and for continuity of the treatment.

The formulations comprising risedronate are usually administered orally and absorbed through the upper gastrointestinal tract. However, the problem of low absorption is encountered in oral administration of risedronate formulations. The absorption of risedronate is reduced by 55% depending on hungriness when taken half an hour before breakfast or two hours after dinner. It has been specified that risedronate maintains its efficiency when it is administered one hour before breakfast; however, absorption is reduced by 30% according to hungriness even in this case.

Another problem which is encountered in oral administration of risedronate is that sufficient bioavailability cannot be ensured depending on low absorption in gastrointestinal system. Although the average bioavailability of risedronate is 0.63%, it is significantly reduced when it is administered with food. For instance, only 0.5% to 5% of the drug is absorbed and only 20% to 50% of the drug which is absorbed shows efficiency by binding to bone tissue in tablet form.

Another factor that affects the bioavailability of risedronate is derived from high gastric acid. Therefore, beverages such as coffee, tea, orange juice reduce the bioavailability.

In addition, oral administration of risedronate and the other bisphosphonates pose irritations in gastrointestinal system. In other words, if the patient takes the drug with less amount of water than required or does not remain in upright position for half an hour after taking the drug, an irritation occurs in gastrointestinal system. These gastrointestinal irritations can be listed as erosion, ulcer and similar irritations in epithelial and mucosal layer of the mouth, buccal cavity, pharynx, larynx and esophagus.

When the prior art is taken into consideration there is need for development of formulations which have higher absorption and therefore higher bioavailability than existing formulations, can overcome the problem of bioavailability reduction due to high gastric acid, and do not cause irritation in gastrointestinal system.

### Description of the Invention

The inventors have surprisingly found that when they formulated the active agent risedronate with an alkaline agent, the formulation obtained had higher absorption and bioavailability as compared to the existing formulations and said formulation does not show bioavailability reduction in high gastric acid and does not cause an irritation in gastrointestinal system.

The alkaline agent used in the formulation is composed of potassium carbonate, potassium bicarbonate, potassium citrate, potassium hydroxide, potassium tartrate, potassium phosphate, sodium carbonate, sodium bicarbonate, sodium citrate, sodium hydroxide, sodium tartrate, sodium phosphate and/or a combination thereof.

When considered from this aspect, the present invention provides a formulation which has high absorption and therefore high bioavailability, can overcome the problem of bioavailability reduction due to high gastric acid, does not cause any irritation in gastrointestinal system therefore provides a formulation which can be used in treatment of bone loss, and diseases associated with bone loss.

The formulation of the present invention comprises the active agent risedronate and one of the following excipients: potassium carbonate, potassium bicarbonate, potassium citrate, potassium hydroxide, potassium tartrate, potassium phosphate, sodium carbonate, sodium bicarbonate, sodium citrate, sodium hydroxide, sodium tartrate, sodium phosphate and/or an alkaline agent selected from binary or multiple combinations thereof.

The alkaline agent used in the formulation of the present invention is preferably sodium carbonate and sodium bicarbonate or a combination thereof.

The amount of alkaline agent comprised in the formulation of the present invention is in the range of 1-90% by weight, preferably in the range of 10-70% by weight, more preferably in the range of 20-50% by weight.

The inventor has seen that the best result is obtained in the formulations comprising risedronate by using sodium carbonate and sodium bicarbonate or a combination thereof in the range of 20-50% by weight in the formulations.

In the case that the formulations of the present invention comprise sodium carbonate and sodium bicarbonate as alkaline agent, the ratio of sodium carbonate to sodium bicarbonate comprised in said formulations is in the range of 0.1 to 3 by weight, preferably in the range of 0.1 to 2 by weight, more preferably in the range of 0.1 to 1 by weight.

The formulations of the present invention comprise alkaline agent and optionally at least one other excipient together with risedronate.

In other words, another characteristic feature of the formulations of the present invention comprising alkaline agent and optionally at least one other excipient together with risedronate is that
- The alkaline agent comprised in the formulations is a combination of sodium carbonate and sodium bicarbonate and
- The weight ratio of sodium carbonate and sodium bicarbonate is in the range of 0.1 to 3, preferably in the range of 0.1 to 2, more preferably in the range of 0.1 to 1.

Risedronate used in the formulation can be in the form of its pharmaceutically acceptable hydrates, solvates, esters, enantiomers, polymorphs, crystalline forms, amorphous forms, salts or free base form and/or a combination thereof.

Risedronate comprised in the formulation of the present invention is in risedronate salt form. These salts are selected from sodium, potassium, calcium, magnesium and ammonium salts; and preferably, sodium salt is used in the formulation of the present invention.

Risedronate comprised in the formulation of the present invention is in hydrate form. These hydrate forms can be selected from monohydrate, dihydrate, trihydrate, hemihydrate, ¼ hydrate, ⅓ hydrate, ²/₃ hydrate, ¾ hydrate, ⁵/₄ hydrate, ⁴/₃ hydrate, ⁵/₂ hydrate and ³/₂ hydrate. Risedronate used in the formulation of the present invention is preferably monohydrate or ⁵/₂ hydrate.

The formulation of the present invention comprises risedronate in the range of 0.1-50 % by weight. The amount of risedronate in the formulation of the present invention is in the range of 5-500 mg, preferably in the range of 10-200 mg.

The formulation of the present invention comprising risedronate is administered orally. Said dosage forms of the present invention that can be administered orally are in solid form such as pill, tablet, capsule, film coated tablet, orodispersible tablet, enterically coated tablet, modified release tablet, prolonged release tablet, delayed release tablet, effervescent powder, effervescent granule, effervescent tablet, sachet, dragee, pastille or liquid form such as suspension, emulsion, syrup, drop, solution; preferably, the formulation is in solid form.

It has been seen that solid dosage forms of the present invention are advantageous in the sense that precise and accurate dose adjustment can be performed and said dosage forms have long shelf life.

Bioavailability of bisphosphonates changes depending on dosage form. For instance, solid dosage forms such as pill, tablet, capsule should firstly disintegrate into small pieces and dissolve in stomach and small intestine in order to be absorbed. Bioavailability is delayed depending on these disintegration and dissolution processes. Furthermore, it has been known that solid dosage forms such as pill, tablet, capsule cause difficulty of use for geriatric, pediatric and disabled patients who have swallowing difficulty. In line with this, the patient fails to comply with drug regimen and this affects efficiency of the treatment and quality of life of the patient.

When the inventors formulate the formulation comprising risedronate and alkaline agent in the form of effervescent powder, granule or tablet; the drug is not exposed to disintegration and dissolution processes since the drug is taken into the body as dissolved in water; therefore, bioavailability is not delayed.

Furthermore, the inventors have provided an easy-to-use formulation for patients when they formulate the formulation comprising risedronate and alkaline agent in the form of effervescent powder, granule or tablet.

The effervescent formulations of the present invention comprising risedronate and alkaline agent comprise at least one pharmaceutically acceptable effervescent acid.

The effervescent acids that can be used in the formulations of the present invention are selected from a group comprising citric acid and acetic acid, tartaric acid, fumaric acid, adipic acid, malic acid or hydrates, anhydrates of these acids or the combinations thereof.

The effervescent formulation of the present invention comprising alkaline agent together with risedronate can optionally comprise one or more of the excipients of binder, lubricant, glidant, diluent, disintegrant, flavouring agent, sweetener, colouring agent, surfactant, antifoaming agent, viscosity agent, stabilizing agent.

The binder mentioned herein can be selected from starches such as potato starch, corn starch, wheat starch; sugars such as sucrose, glucose, dextrose, lactose, maltodextrin; natural and synthetic gums; gelatine; cellulose derivatives such as microcrystalline cellulose, HPC, HEC, HPMC, carboxymethylcellulose, methylcellulose, ethylcellulose; polyvinylpyrrolidone (povidone); polyethylene glycol (PEG); waxes; calcium carbonate; calcium phosphate; alcohols such as sorbitol, xylitol, mannitol and water or a combination thereof.

The lubricant mentioned herein can be selected from sodium lauryl sulphate, sodium benzoate, sodium chloride, sodium acetate, sodium fumarate, carbowax 4000, L- leucine (17), polyethylene glycol (PEG) or a combination thereof.

The amount of the lubricant used in the formulation is in the range of 0.1-5%. It has been seen that PEG used in said amount is effective in providing efficient lubrication and preventing the active agent from generating metal complexes with the devices which have metal surfaces during production.

The lubricant used in the formulation of the present invention is polyethylene glycol (PEG).

In other words, another characteristic feature of the formulations of the present invention comprising alkaline agent and optionally at least one other excipient together with risedronate is that
- The alkaline agent comprised in the formulations is a combination of sodium carbonate and sodium bicarbonate,
- The weight ratio of sodium carbonate and sodium bicarbonate is in the range of 0.1 to 3, preferably in the range of 0.1 to 2, more preferably in the range of 0.1 to 1 and
- The formulation comprises polyethylene glycol in the range of 0.1-5% by weight as the lubricant.

The glidant mentioned herein can be selected from talc, magnesium stearate, stearic acid, sodium stearyl fumarate, polyoxyethylene glycol, leucine, alanine, glycine, sodium benzoate, sodium acetate, fumaric acid or a combination thereof.

The diluent mentioned herein can be selected from lactose, maltose, dextrin, maltodextrin, mannitol, sorbitol, starch or a combination thereof.

The diluent used in the formulation of the present invention is maltodextrin. The amount of the diluent used in the formulation is in the range of 1-10% by weight.

The disintegrant mentioned herein can be selected from starches such as potato starch, corn starch, wheat starch, pregelatinized starch, sodium starch glycolate; cellulose derivatives such as croscarmellose sodium or microcrystalline cellulose; polyvinylpyrrolidone; crospovidone; alginic acid and its salts; chyles such as xanthan gum and veegum; ion exchange resins or a combination thereof.

The flavouring agent mentioned herein can be selected from natural aroma oils (such as peppermint oil, oil of wintergreen, clove bud oil, parsley oil, eucalyptus oil, lemon oil, orange oil), menthol, menthane, anethole, methyl salicylate, eucalyptol, cinnamon, 1- methyl acetate, sage, eugenol, oxanon, alpha-irisone, marjoram, lemon, orange, blackberry, propenyl guaetol acetyl, cinnamon, vanilla, timole, linalol, cinnamaldehyde glycerol acetal, N-substituted p-menthane-3-carboxamide, 3,1-methoxy propane 1.2-diol or a combination thereof.

The sweetener mentioned herein can be selected from sucralose, sucrose, fructose, glucose, galactose, xylose, dextrose, laevulose, lactose, maltose, maltodextrin, mannitol, maltitol, maltol, sorbitol, xylitol, erythritol, lactitol, isomalt, corn syrup, saccharine, saccharine salts, acesulphame potassium, aspartame, D-tryptophan, monoammonium glycyrrhizinate, neohesperidin dihydrochalcone, thaumatin, neotame, alitame, stevioside and cyclamates or a combination thereof.

The colouring agent mentioned herein can be selected from carotenoids and chlorophyll or a combination thereof.

The surfactant mentioned herein can be selected from sodium lauryl sulphate and magnesium lauryl sulphate or a combination thereof.

The antifoaming agent mentioned herein can be selected from simethicone emulsion and dimethyl siloxane, silicone oil or a combination thereof.

The viscosity agent mentioned herein can be selected from carboxymethyl cellulose, methyl cellulose, xanthan gum, gummi tragacanthae, gum arabic, aerosil 200, colloidon, agar-agar, bentonite, hydroxyethyl cellulose or a combination thereof.

The stabilizing agent and/or agents mentioned herein can be selected from agents such as antioxidants, chelating agents, alkalinizing agents and photo protective agents.

The antioxidants can be selected from substances such as butylated hydroxyanisole (BHA), sodium ascorbate, butylated hydroxytoluene (BHT), sodium sulphite, gallates (such as propyl gallate), tocopherol, citric acid, malic acid, ascorbic acid, acetylcysteine, fumaric acid, lecithin, ascorbyl palmitate, ethylenediamine tetraacetate or a combination thereof.

The chelating agents can be selected from disodium EDTA, edetic acid, citric acid, sodium citrate, potassium citrate or a combination thereof.

The alkalizing agents can be selected from alkali metal salts such as sodium carbonate, sodium hydrogen carbonate, sodium hydroxide, sodium silicate, disodium hydrogen ortophosphate, sodium aluminate; earth alkali metal salts such as calcium carbonate, calcium hydroxide, dibasic calcium phosphate, tribasic calcium phosphate, calcium sulphate, calcium acetate, calcium gluconate, calcium glycerophosphate, magnesium carbonate, magnesium hydroxide, magnesium sulphate, magnesium acetate, magnesium silicate, magnesium aluminate and organic compounds such as primary, secondary and tertiary amines, cyclic amines, N-N'-dibenzylethylenediamine, diethanoleamine, ethylenediamine, meglumine, monosodium glutamate, polyacryline sodium, sodium alginate or a combination thereof.

The photo-protective agents can be selected from metal oxides such as titanium oxide, iron oxide or zinc oxide or a combination thereof.

The formulation of the present invention can preferably be combined with the other active agent.

The term "other active agent" mentioned herein refers to various vitamins and/or minerals required for human body.

The dosage forms can be taken separately, simultaneously and sequently for combined therapy, as well as taken as a combination of risedronate with the other active agent or agents in a single dosage form.

The other active agent or agents that can be used with risedronate in combined therapy can be minerals such as calcium, potassium, magnesium, iron, sodium, zinc or salts thereof such as carbonate, sulphate; vitamins such as vitamin A, B vitamins such as B1, B12, B6 and/or folic acid; vitamin C, vitamin D and its analogs (such as Vitamin D2 (ergocalciferol), Vitamin D3 (cholecalciferol)), vitamin E. The second active agent which is preferred is vitamin D and/or calcium.

One or two of the other active agents listed above can be combined with risedronate in combined therapy. In other words, the present invention refers to binary and ternary combinations of risedronate with the other active agents specified above.

The other active agent or agents that can be used in combined therapy can be produced with risedronate and by the same production method, as well as prepared by producing the active agent formulations separately and combining them afterwards.

The formulation of the present invention can be used in treatment of osteoporosis; in order to reduce risk of bone fracture in women including spine bone and hipbone; in order to reduce risk of bone fracture in men; in treatment of diseases such as idiopathic osteoporosis; osteoporosis resulting from various diseases; osteoporosis resulting from steroid and glucocorticoid; osteopenia, osteomalacia, osteogenesis imperfecta, osteochondrodysplasia, sudeck atrophy, rheumatoid arthritis, Paget's disease, bone metastasis of malignant tumors, hypercalcaemia or hyperthyroidism; and particularly in adolescence, pregnant or nursing women in premenopausal and postmenopausal period in order to preserve bone health as nutrition reinforcement.

The examples required in order to render the formulation of the present invention more comprehensible are given below. Yet, the subject of the present invention should not be limited to these examples.

### EXAMPLES

### Example 1

The granulation solution is prepared by mixing the binder and the diluent. Risedronate, sodium carbonate and sodium bicarbonate are mixed in fluid bed and granulated with the granulation solution prepared. The granules obtained are mixed with the lubricant and the other excipients. The formulation prepared can be packed in the form of granule and also the formulation can be compressed in tablet form.

| **Component** | **Amount (% by weight)** |
|---|---|
| Risedronate sodium | 2 |
| Sodium bicarbonate | 30 |
| Sodium carbonate | 10 |
| Citric acid | 50 |
| Polyethylene glycol | 2 |
| Diluent | 3.5 |
| Binder | 1.5 |
| Other excipients | 1 |
| **Total** | **100** |

### Example 2

| **Component** | **Amount (% by weight)** |
|---|---|
| Risedronate sodium | 5 |
| Sodium bicarbonate | 35 |
| Citric acid | 50 |
| Polyethylene glycol | 1 |
| Other excipients | 9 |
| **Total** | **100** |

## Claims

1. A formulation comprising risedronate and/or a pharmaceutically acceptable derivative thereof **characterized in that** said formulation comprises
• sodium carbonate and sodium bicarbonate as alkaline agent wherein the ratio of sodium carbonate to sodium bicarbonate is in the range of 0.1 to 3 by weight and
• optionally at least one pharmaceutically acceptable excipient.

2. The formulation according to claim 1 **characterized in that** the ratio of sodium carbonate to sodium bicarbonate is in the range of 0.1 to 2 by weight.

3. The formulation according to claims 1-2 **characterized in that** the ratio of sodium carbonate to sodium bicarbonate is in the range of 0.1 to 1 by weight.

4. The formulation according to claims 1-3 **characterized in that** the amount of alkaline agent comprised in said formulation is in the range of 1-90% by weight.

5. The formulation according to claim 4 **characterized in that** the amount of alkaline agent comprised in said formulation is in the range of 10-70% by weight.

6. The formulation according to claim 5 **characterized in that** the amount of alkaline agent comprised in said formulation is in the range of 20-50% by weight.

7. The formulation according to claim 1 **characterized in that** risedronate comprised in said formulation is in the form of its pharmaceutically acceptable hydrates, solvates, esters, enantiomers, polymorphs, crystalline forms, amorphous forms, salts or free base form and/or a combination thereof.

8. The formulation according to any preceding claims **characterized in that** the amount of risedronate comprised in said formulation is in the range of 0.1-50% by weight.

9. The formulation according to claim 8 **characterized in that** the amount of risedronate comprised in said formulation is in the range of 5-500 mg.

10. The formulation according to claim 9 **characterized in that** the amount of risedronate comprised in said formulation is in the range of 10-200 mg.

11. The formulation according to any preceding claims **characterized in that** said formulation optionally comprises one or more of the excipients of binder, lubricant, glidant, diluent, disintegrant, flavouring agent, sweetener, colouring agent, surfactant, antifoaming agent, viscosity agent, stabilizing agent.

12. The formulation according to claim 11 **characterized in that** said formulation comprises a lubricant selected from a group comprising sodium lauryl sulphate, sodium benzoate, sodium chloride, sodium acetate, sodium fumarate, carbowax 4000, L-leucine(17), polyethylene glycol (PEG) or a combination thereof.

13. The formulation according to claim 12 **characterized in that** said formulation comprises a lubricant in the range of 0.1-5% by weight selected from a group comprising sodium lauryl sulphate, sodium benzoate, sodium chloride, sodium acetate, sodium fumarate, carbowax 4000, L-leucine(17), polyethylene glycol (PEG) or a combination thereof.

14. The formulation according to claim 13 or 14 **characterized in that** said formulations comprise polyethylene glycol (PEG) in the range of 0.1-5% by weight as the lubricant.

## Patentansprüche

1. Eine Formulierung, die Risedronat und / oder ein pharmazeutisch verträgliches Derivat davon beinhaltet, **dadurch gekennzeichnet, dass** die genannte Formulierung die Folgenden umfasst:
• Natriumcarbonat und Natriumbicarbonat als alkalisches Mittel, wobei das Verhältnis von Natriumcarbonat zu Natriumbicarbonat im Bereich von 0,1 bis 3 Gewichtsprozent liegt und
• gegebenenfalls mindestens einen pharmazeutisch annehmbaren Hilfsstoff.

2. Die Formulierung nach Anspruch 1, **dadurch gekennzeichnet, dass** das Verhältnis von Natriumcarbonat zu Natriumbicarbonat im Bereich von 0,1 bis 2 Gewichtsprozent liegt,

3. Die Formulierung nach den Ansprüchen 1-2, **dadurch gekennzeichnet, dass** das Verhältnis von Natriumcarbonat zu Natriumbicarbonat im Bereich von 0,1 bis 1 Gewichtsprozent liegt.

4. Die Formulierung nach den Ansprüchen 1-3, **dadurch gekennzeichnet, dass** die Menge des alkalischen Mittels, das in der Formulierung erhältlich ist, im Bereich von 1-90 Gewichtsprozent liegt.

5. Die Formulierung nach Anspruch 4, **dadurch gekennzeichnet, dass** die Menge des alkalischen Mittels, das in der Formulierung erhältlich ist, im Bereich von 10-70 Gewichtsprozent liegt,

6. Die Formulierung nach Anspruch 5, **dadurch gekennzeichnet, dass** die Menge des alkalischen Mittels, das in der Formulierung erhältlich ist, im Bereich von 20-50 Gewichtsprozent liegt.

7. Die Formulierung nach Anspruch 1, **dadurch gekennzeichnet, dass** Risedronat, das in der Formulierung erhältlich ist, in Form seiner pharmazeutisch annehmbaren Hydrate, Solvate, Ester, Enantiomere, Polymorphe, kristallinen Formen, amorphen Formen, Salze oder freien Base und / oder einer Kombination davon liegt.

8. Die Formulierung nach einem der vorhergehenden Ansprüche, **dadurch gekennzeichnet, daß** die Menge von Risedronat, das in der Formulierung erhältlich ist, im Bereich von 0,1-50 Gewichtsprozent liegt.

9. Die Formulierung nach Anspruch 8, **dadurch gekennzeichnet, dass** die Menge von Risedronat, das in der Formulierung erhältlich ist, im Bereich von 5-500 mg liegt.

10. Die Formulierung nach Anspruch 9, **dadurch gekennzeichnet, dass** die Menge von Risedronat, das in der Formulierung erhältlich ist, im Bereich von 10-200 mg liegt,

11. Die Formulierung nach einem der vorhergehenden Ansprüche, **dadurch gekennzeichnet, daß** die genannte Formulierung gegebenenfalls ein oder mehrere der Hilfsstoffe aus Bindemittel, Schmiermittel, Gleitmittel, Verdünnungsmittel, Sprengmittel, Geschmacksmittel, Sßungsmittel, Färbemittel, Tensid, Antischaummittel, Viskositätsmittel, Stabilisierungsmittel beinhaltet.

12. Die Formulierung nach Anspruch 11, **dadurch gekennzeichnet, dass** die genannte Formulierung ein Schmiermittel enthält, das aus einer Gruppe enthaltend Natriumlaurylsulfat, Natriumbenzoat, Natriumchlorid, Natriumacetat, Natriumfumarat, Carbowax 4000, L-Leucin (17), Polyethylenglykol (PEG) oder eine Kombination davon ausgewählt wird.

13. Die Formulierung nach Anspruch 12, **dadurch gekennzeichnet, daß** die genannte Formulierung ein Gleitmittel im Bereich von 0,1-5 Gewichtsprozent enthält, das aus einer Gruppe enthaltend Natriumlaurylsulfat, Natriumbenzoat, Natriumchlorid, Natriumacetat, Natriumfumarat, Carbowax 4000, L-Leucin (17), Polyethylenglycol (PEG) oder eine Kombination davon ausgewählt wird,

14. Die Formulierung nach Anspruch 13 oder 14, **dadurch gekennzeichnet, dass** die genannten Formulierungen Polyethylenglykol (PEG) im Bereich von 0,1-5 Gewichtsprozent als Schmiermittel enthält,

## Revendications

1. Une formulation comprenant le risédronate et/ou un dérivé de celui-ci pharmaceutiquement acceptable, **caractérisée en ce que** ladite formulation comprend
• le carbonate de sodium et le bicarbonate de sodium comme agent alcalin dans lequel le rapport entre le carbonate de sodium et le bicarbonate de sodium est dans la plage de 0,1 à 3 en poids, et
• éventuellement au moins un excipient pharmaceutiquement acceptable.

2. La formulation selon la revendication 1, **caractérisée en ce que** le rapport entre le carbonate de sodium et le bicarbonate de sodium est dans la plage de 0,1 à 2 en poids.

3. La formulation selon les revendications 1-2, **caractérisée en ce que** le rapport entre le carbonate de sodium et le bicarbonate de sodium est dans la plage de 0,1 à 1 en poids.

4. La formulation selon les revendications 1-3, **caractérisée en ce que** la quantité de l'agent alcalin comprise dans ladite formulation est dans la plage de 1-90% en poids.

5. La formulation selon la revendication 4, **caractérisée en ce que** la quantité de l'agent alcalin comprise dans ladite formulation est dans la plage de 10-70% en poids.

6. La formulation selon la revendication 5, **caractérisée en ce que** la quantité de l'agent alcalin comprise dans ladite formulation est dans la plage de 20-50% en poids.

7. La formulation selon la revendication 1, **caractérisée en ce que** le risédronate compris dans ladite formulation est dans la forme de ses hydrates, ses solvates, ses esters, ses énantiomères, ses polymorphes, ses formes cristallines, ses formes amorphes, ses formes d'un sel ou d'un base libres pharmaceutiquement acceptables et/ou d'une combinaison de ceux-ci.

8. La formulation selon l'une quelconque des revendications précédentes, **caractérisée en ce que** la quantité du risédronate comprise dans ladite formulation est dans la plage de 0,1-50% en poids.

9. La formulation selon la revendication 8, **caractérisée en ce que** la quantité du risédronate comprise dans ladite formulation est dans la plage de 5-500 mg.

10. La formulation selon la revendication 9, **caractérisée en ce que** la quantité du risédronate comprise dans ladite formulation est dans la plage de 10-200 mg.

11. La formulation selon l'une quelconque des revendications précédentes, **caractérisée en ce que** ladite formulation comprend éventuellement un ou plusieurs des excipients parmi le liant, le lubrifiant, le glissant, le diluant, le désintégrant, l'agent aromatisant, l'édulcorant, l'agent colorant, l'agent tensioactif, l'agent antimousse, l'agent de viscosité, l'agent stabilisant.

12. La formulation selon la revendication 11, **caractérisée en ce que** ladite formulation comprend un lubrifiant choisi d'un groupe comprenant le laurylsulfate de sodium, le benzoate de sodium, le chlorure de sodium, l'acétate de sodium, le fumarate de sodium, le Carbowax 4000, L-leucine (17), le polyéthylène glycol (PEG) ou une combinaison de ceux-ci.

13. La formulation selon la revendication 12, **caractérisée en ce que** ladite formulation comprend un lubrifiant dans la plage de 0,1-5% en poids choisi d'un groupe comprenant le laurylsulfate de sodium, le benzoate de sodium, le chlorure de sodium, l'acétate de sodium, le fumarate de sodium, le Carbowax 4000, L-leucine (17), le polyéthylène glycol (PEG) ou une combinaison de ceux-ci.

14. La formulation selon la revendication 13 ou 14, **caractérisée en ce que** lesdites formulations comprennent le polyéthylène glycol (PEG) dans la plage de 0.1-5% en poids comme le lubrifiant.
